(19) **Europäisches Patentamt**
**European Patent Office**
**Office européen des brevets**

(11) **EP 1 726 632 B1**

(12) **EUROPEAN PATENT SPECIFICATION**

(45) Date of publication and mention
of the grant of the patent:
**25.04.2012 Bulletin 2012/17**

(51) Int Cl.:
**C09K 15/08** (2006.01)    **A23L 3/3508** (2006.01)
**C09K 15/34** (2006.01)

(21) Application number: **05720993.4**

(86) International application number:
**PCT/JP2005/004806**

(22) Date of filing: **17.03.2005**

(87) International publication number:
**WO 2005/090518 (29.09.2005 Gazette 2005/39)**

(54) **DEGRADATION INHIBITOR**

ABBAUINHIBITOR

INHIBITEUR DE DEGRADATION

(84) Designated Contracting States:
**AT BE BG CH CY CZ DE DK EE ES FI FR GB GR HU IE IS IT LI LT LU MC NL PL PT RO SE SI SK TR**

(30) Priority: **19.03.2004 JP 2004080224**

(43) Date of publication of application:
**29.11.2006 Bulletin 2006/48**

(60) Divisional application:
**10009488.7 / 2 261 304**

(73) Proprietor: **Mitsubishi Chemical Corporation Tokyo 108-0014 (JP)**

(72) Inventor: **KIDO, Hirotsugu,**
**MITSUBISHI CHEMICAL CORPORATION**
**Yokohama-shi,**
**Kanagawa 2278502 (JP)**

(74) Representative: **Woods, Geoffrey Corlett**
**J A Kemp**
**14 South Square**
**Gray's Inn**
**London WC1R 5JJ (GB)**

(56) References cited:
**EP-A- 1 344 516      WO-A-00/39066**
**WO-A-02/15860      WO-A-97/06697**
**WO-A-03/011234      WO-A-2004/020552**
**JP-A- 8 253 764      JP-A- 2001 342 489**
**JP-A- 2002 142 673   US-A- 5 084 289**
**US-A- 5 084 293      US-A- 5 314 686**
**US-A- 5 795 609      US-A- 5 919 483**
**US-A- 6 066 327**

## EP 1 726 632 B1

**Description**

<u>TECHNICAL FIELD</u>

The present invention relates to an anti-degradation agent, and more particularly, to an anti-degradation agent which is also useful as an agent for improving agent a keeping property of various products themselves such as foods and cosmetics.

<u>BACKGROUND ART</u>

Deterioration of substances is caused by a quality change as well as a reaction between components thereof in association with the quality change. These change and reaction are caused at the same time or in a chain-like or serial manner. By a result of studies until now, these deterioration processes relate to oxidation or photo-deterioration. The oxidation or the photo-deterioration is caused under such circumstances in air, in water, at an interface between air and water, at an interface between water and oil or at an interface between air and oil. The factors for accelerating the oxidative deterioration include enzymes, metals and sensitizers. The photo-deterioration is caused when the substances absorb ultraviolet ray, visible light or near-infrared ray. From these, the deterioration of substances is caused owing to combination of these factors.

The beverage or food and the perfume or cosmetic tend to be usually deteriorated by oxidation of perfume components, pigments or other materials blended therein during the production process or storage thereof. It is important to prevent such an oxidation for the purpose of maintaining a good quality of each of the beverage or food and the perfume or cosmetic. For this reason, in the beverage or food and the perfume or cosmetics, there have been used, for example, natural antioxidants, synthetic antioxidants or preparations obtained by appropriately blending these antioxidants with each other (hereinafter totally referred to merely as "antioxidants").

For example, there are generally known antioxidants made of rosmarinic acid, anti-degradation agents made of carnosol or carnosic acid, antioxidants made of herbs such as rosemary, and antioxidants containing vitamin C or vitamin E (e.g., refer to Patent Documents 1 and 2). In addition, there are also known anti-degradation agents containing rosmarinic acid, carnosol and carnosic acid as rosemary extracts. However, among these rosemary extracts, the rosmarinic acid is water-soluble, whereas the carnosol and carnosic acid are water-insoluble. Therefore, if it is intended to prepare such an anti-degradation agent containing all three kinds of the rosemary extracts, the contents of the respective extracts as well as the balance therebetween in the anti-degradation agent can be controlled only to a limited extent owing to the extraction conditions (in particular, a solvent used). More specifically, the content of rosmarinic acid in the anti-degradation agent is about 2% by weight at most, whereas the total content of the carnosol and carnosic acid is about 1% by weight at most. In addition, these antioxidants tend to be unsatisfactory in stability of an oxidation-inhibiting performance thereof against outside environmental conditions.

In particular, in application fields such as food and perfume or cosmetic, it has been strongly required to provide antioxidants which can exhibit a good effect even when added in a small amount and are free from deterioration even upon heating. However, at present, no antioxidants fully satisfying the above requirements are known in the art until now. Further, since the photo-deterioration and the oxidative deterioration are frequently different in mechanism, etc., from each other, the antioxidants may fail to exhibit a satisfactory effect as a photo-deterioration inhibitor even though they are effective as an oxidation preventing agent. In addition, the antioxidants might sometimes undergo photo-deterioration by themselves and, therefore, may fail to exhibit a fully satisfactory effect as the photo-deterioration inhibitor. US 5 795 609 and JP 8 253 764 describe a method for obtaining antioxidant substances from vegetable matter by mixing vegetable matter containing antioxidant substances with a $C_2$-$C_6$ alkylene glycol and subjecting the mixture to a pressure of at least 40 bar.

Patent Document 1: Japanese Patent Application Laid-open (KOKAI) No. 2002-363557
Patent Document 2: Japanese Patent Application Laid-open (KOKAI) No. 2003-55686

<u>DISCLOSURE OF THE INVENTION</u>

<u>Problem to be Solved by the Invention</u>

The present invention has been made in view of the above conventional problems. An object of the present invention is to provide an anti-degradation agent which has an excellent anti-degradation performance for food, cosmetic, etc., and can exhibit a good effect even when added in a small amount, show a high heat resistance, and is free from adverse influence due to light.

Means for Solving Problem

As a result of the present inventors' earnest study for solving the above conventional problems, it has been found that the above object can be achieved by such a composition containing a water-soluble antioxidant and a water-insoluble antioxidant which may further contain an emulsifying agent. In addition, it has also been found that these compositions exhibit common properties defined by predetermined parameters.

The present invention has been attained on the basis of the above findings. The present invention is constituted from a plurality of aspects mentioned below:

(i) An anti-degradation agent which comprises: a water-insoluble antioxidant having a solubility in 100 g of water of less than 0.1 g at 25 °C, wherein the water insoluble antioxidant comprises carnosol and/or carnosic acid and the total amount of the carnosol and/or carnosic acid in the anti-degradation agent is not less than 4% by weight; and a water-soluble antioxidant having a solubility in 100 g of water of not less than 0.1 g at 25 °C; and which anti-degradation agent has a photo resistance of not less than 10 and a heat resistance of not less than 10 wherein the photo resistance is determined as in definition (A) and the heat resistance as in definition (B) or the photo resistance is determined as in definition (C) and the heat resistance as in definition (D);

(A) When a food as a sample is placed in a container and irradiated with light at a total illuminance of 500,000 lux to measure an amount of hexanal generated from the food and accumulated within a head space of the container by gas chromatography, said photo resistance being calculated from the following formula (1):

$$\text{Photo resistance} = [\{(\text{Amount of hexanal generated from the light-irradiated food containing no anti-degradation agent}) - (\text{Amount of hexanal generated from the light-irradiated food containing the anti-degradation agent})\}/\{(\text{Amount of hexanal generated from the light-irradiated food containing no anti-degradation agent}) - (\text{Amount of hexanal generated from the light-non-irradiated food containing the anti-degradation agent})\}] \times 100 \cdots (1);$$

(B) When a food as a sample is placed in a container, air is blown into the container, and the food is maintained at a temperature of 60°C to measure a time at which an amount of volatile components generated from the food is rapidly increased (degradation induction time) using an electrical conductivity meter, said heat resistance being calculated from the following formula (2):

$$\text{Heat Resistance} = [\{(\text{Degradation induction time of the food containing the anti-degradation agent upon heating}) - (\text{Degradation induction time of the food containing no anti-degradation agent upon heating})\}/(\text{Degradation induction time of the food containing no anti-degradation agent upon heating})] \times 100 \cdots (2);$$

(C) When a solution of a pigment as a sample filled in a container is irradiated with light at a total illuminance of 500,000 lux to determine a degree of discoloration of the pigment by measuring an absorbance of the pigment solution at a specific wavelength, said photo resistance being calculated from the following formula (3):

$$\text{Photo resistance} = [\{(\text{Absorbance of the light-irradiated pigment solution containing the anti-degradation agent}) - (\text{Absorbance of the light-irradiated pigment solution containing no anti-degradation agent})\}/\{(\text{Absorbance of the light-non-irradiated pigment solution containing the anti-degradation agent}) - (\text{Absorbance of the light-irradiated pigment solution containing no anti-degradation agent})\}] \times 100 \cdots (3);$$

and
(D) When a pigment as a sample placed in a container is held at 55°C for one week to determine a degree of discoloration of the pigment by measuring an absorbance of a solution of the pigment at a specific wavelength, said heat resistance being calculated from the following formula (4):

$$\text{Heat Resistance} = [\{(\text{Absorbance of the heated pigment solution containing the anti-degradation agent}) - (\text{Absorbance of the heated pigment solution containing no anti-degradation agent})\}/\{(\text{Absorbance of the non-heated pigment solution containing the anti-degradation agent}) - (\text{Absorbance of the heated pigment solution containing no anti-degradation agent})\}] \times 100 \cdots (4).$$

(ii) A beverage or food comprising the anti-degradation agent as defined in (i) in an amount of 0.0001 to 30% by weight.
(iii) A diet or pet food comprising the anti-degradation as defined in (i) in an amount of 0.0001 to 30% by weight.
(iv) A perfume or cosmetic comprising the anti-degradation agent as defined in (i) in an amount of 0.0001 to 30% by weight.
(v) A glaze agent comprising the anti-degradation agent as defined in (i) in an amount of 0.0001 to 30% by weight.
(vi) A plastic product comprising the anti-degradation agent as defined in (i) in an amount of 0.00001 to 20% by weight.
(vii) A process which comprises applying to a beverage or food the anti-degradation agent as defined in (i).
(viii) A process which comprises applying to a diet or pet food the anti-degradation agent as defined in (i).
(ix) A process which comprises applying to a perfume or cosmetic the anti-degradation agent as defined in (i).
(x) A process which comprises applying to a glaze agent the anti-degradation agent as defined in (i).
(xi) A process which comprises applying to a plastic product the anti-degradation agent as defined in (i).

EFFECT OF THE INVENTION

The anti-degradation agent of the present invention has a high safety, is usable even in a small amount, exhibits a high heat resistance, and is excellent in oxidative deterioration preventing property and photo-deterioration preventing property for foods, cosmetics, etc.

PREFERRED EMBODIMENTS FOR CARRYING OUT THE INVENTION

The present invention is described in detail below.

<Anti-degradation agent>

First, the anti-degradation agent according to the first aspect of the present invention is explained. The anti-degradation agent has a photo resistance of not less than 10 and a heat resistance of not less than 10 which can in one aspect be determined by the following definitions (A) and (B):

(A) when a food as a sample is placed in a container and irradiated with light at a total illuminance of 500,000 lux to measure an amount of hexanal generated from the food and accumulated within a head space of the container by gas chromatography, said photo resistance being calculated from the following formula (1):

$$\text{Photo resistance} = [\{(\text{Amount of hexanal generated from the light-irradiated food containing no anti-degradation agent}) - (\text{Amount of hexanal generated from the light-irradiated food containing the anti-degradation agent})\}/\{(\text{Amount of hexanal generated from the light-irradiated food containing no anti-degradation agent}) - (\text{Amount of hexanal generated from the light-non-irradiated food containing the anti-degradation agent})\}] \times 100 \cdots (1);$$

and
(B) when a food as a sample is placed in a container, air is blown into the container, and the food is maintained at a temperature of 60°C to measure a time at which an amount of volatile components generated from the food is rapidly increased (degradation induction time) using an electrical conductivity meter, said heat resistance being calculated from the following formula (2):

$$\text{Heat Resistance} = [\{(\text{Degradation induction time of the food containing the anti-degradation agent upon heating}) - (\text{Degradation induction time of the food containing no anti-degradation agent upon heating})\}/(\text{Degradation induction time of the food containing no anti-degradation agent upon heating})] \times 100 \cdots (2).$$

The photo resistance and the heat resistance are evaluation parameters adopted on the basis of the following facts. That is, among ordinary deterioration phenomena, in those due to thermal degradation, rapid generation of volatile components tends to be caused when exceeding a certain threshold value of time, resulting in occurrence of rapid deterioration. For this reason, in the present invention, the threshold value of time is utilized as a degradation induction time. In the photo-deterioration, oils and fats or proteins used in foods tend to undergo deterioration when irradiated with light, so that fatty acids or amino acids contained therein are decomposed to produce an aldehyde. At this time, the amount of hexanal which has a low threshold value of time and is contained in a large amount in the deteriorated foods, is measured to determine the change thereof between before and after the light irradiation, and is utilized to evaluate a photo-deterioration preventing property, i.e., a photo resistance.

In another aspect, the photo resistance of not less than 10 and the heat resistance of not less than 10 are respectively determined by the following definitions (C) and (D). The anti-degradation agent can in this aspect be suitably applied, in particular, to objectives containing a pigment.

(C) When a solution of a pigment as a sample filled in a container is irradiated with light at a total illuminance of 500,000 lux to determine a degree of discoloration of the pigment by measuring an absorbance of the pigment solution at a specific wavelength, said photo resistance being calculated from the following formula (3):

Photo resistance = [{(Absorbance of the light-irradiated pigment solution containing the anti-degradation agent) - (Absorbance of the light-irradiated pigment solution containing no anti-degradation agent)}/{(Absorbance of the light-non-irradiated pigment solution containing the

anti-degradation agent) - (Absorbance of the light-irradiated pigment solution containing no anti-degradation agent)}] x 100 ··· (3);

and
(D) when a pigment as a sample placed in a container is held at 55°C for one week to determine a degree of discoloration of the pigment by measuring an absorbance of a solution of the pigment at a specific wavelength, said heat resistance being calculated from the following formula (4):

Heat Resistance = [{(Absorbance of the heated pigment solution containing the anti-degradation agent) - (Absorbance of the heated pigment solution containing no anti-degradation agent)}/{(Absorbance of the non-heated pigment solution containing the anti-degradation agent) - (Absorbance of the heated pigment solution containing no anti-degradation agent)}] x 100 ··· (4).

As foods serving for measuring the photo resistance and heat resistance of the anti-degradation agent, there may be usually used a mixture of 30 parts by weight of soybean oil, 50 parts by weight of wheat flour and 20 parts by weight of water. Upon the respective measurements, there may be used two kinds of test specimens, i.e., one specimen prepared by mixing the above mixture with 0.1 part by weight of the anti-degradation agent and mechanically kneading these components with each other, and the other specimen prepared by mechanically kneading the above mixture solely without adding no anti-degradation agent thereto. Also, in the above definitions, the light with a total illuminance of 500,000 lux may be achieved, for example, by irradiating light with an illuminance of 20,000 lux for 25 hr. The time at which the amount of volatile components is rapidly increased (degradation induction time) may be determined by detecting generation of volatile components using an electrical conductivity meter (e.g., "RANCIMAT 743 MODEL" manufactured by Metronome Inc.).
The anti-degradation agent of the present invention preferably has a photo resistance of not less than 20 and a heat resistance of not less than 20. More specifically, the anti-degradation agent of the present invention can be realized by such anti-degradation agents containing a water-insoluble antioxidant and a water-soluble antioxidant. These components are described in detail hereinafter. Further, the present invention can be achieved by the below-mentioned anti-degradation agent (II).

According to another aspect of the present invention the anti-degradation agent contains a water-insoluble antioxidant, a water-soluble antioxidant and an emulsifying agent.

(Water-insoluble antioxidant)

The water-insoluble antioxidant used in the present invention means an antioxidant having a solubility in 100 g of water of less than 0.1 g, preferably not more than 0.05 g and more preferably not more than 0.01 g as measured at 25°C. Examples of water-insoluble antioxidant may include tea extracts, catechin, epicatechin, epigallocatechin, catechin gallate, epigallocatechin gallate, vitamin E ($\alpha$, $\beta$, $\gamma$, $\delta$-tocopherol), mixed tocopherol and vitamin C fatty esters. Further, the water-soluble antioxidant comprises carnosol and/or carnosic acid. The details of the carnosol and carnosic acid are described hereinlater.

(Water-soluble antioxidant)

The water-soluble antioxidant used in the present invention means an antioxidant having a solubility in 100 g of water of not less than 0.1 g, preferably not less than 0.5 g, more preferably not less than 1 g and still more preferably not less than 5 g as measured at 25°C. Examples of the water-soluble antioxidant may include water-soluble natural extracts such as water-soluble rosemary extracts, and vitamin C.

Rosmarinic acid is one of phenol-carboxylic acids contained in herbs, in particular, contained in a large amount in rosemary. Rosmarinic acid has such a structure in which two phenol-carboxylic acids are bonded to each other. Therefore, rosmarinic acid structurally and functionally exhibits a higher oxidation-inhibiting effect than those of phenol-carboxylic acids such as ferulic acid, caffeic acid and chlorogenic acid because of a larger number of phenolic hydroxyl groups contained therein. Further, rosmarinic acid exhibits a high activation effect for inhibition of enzyme reaction like SOD (superoxide dimustase). In addition, rosmarinic acid also has a high photo-deterioration inhibiting effect because of conjugated double bond contained in a structure thereof.

Rosmarinic acid used in the present invention is preferably in the form of a natural extract and more preferably a glycoside formed by bonding a sugar to the rosmarinic acid from the viewpoint of safety. Thus, the rosmarinic acid used in the present invention also includes the glycosides of rosmarinic acid. The glycosides having any structure may be used in the present invention. Natural products of rosmarinic acid may be extracts obtained from herbs, in particular, lamiaceous plants and preferably extracts obtained from rosemary containing a large amount of rosmarinic acid.

The general production method of rosmarinic acid is as follows. As the raw material, there may be used a whole grass of rosemary, or any of leaves, roots, stems, flowers, fruits and seeds of rosemary. Among them, preferred are leaves of rosemary. In order to enhance the extraction efficiency, rosemary may be usually used in the form of cut pieces. The rosmarinic acid is obtained as a water-soluble extract of rosemary. Therefore, the rosmarinic acid may be produced by subjecting rosemary to extraction treatment with hexane, hexane/ethanol, ethanol, hydrated ethanol or supercritical carbon dioxide, adding water to the resultant extract to precipitate water-insoluble components therefrom, and concentrating the obtained solution from which the water-insoluble components are removed, under reduced pressure. As the hydrated ethanol, there may be preferably used those having a water content of 40 to 60% by weight.

(Carnosol and carnosic acid)

The carnosol and carnosic acid are contained in a large amount in not only rosemary but also herb-based condiments such as sage, thyme and oregano. The carnosol and carnosic acid have an abietane structure containing an isoprene skeleton unlike the other antioxidants and, therefore, exhibit a considerably high oxidation-inhibiting effect on fats and oils, etc., as compared to the other antioxidants. In addition, the carnosol and carnosic acid have a conjugated double bond in a structure thereof and further a tautomerism structure. Therefore, the carnosol and carnosic acid tend to be structurally stabilized against radicals even when undergoing influence of the radicals and, as a result, can exhibit a high photo-deterioration inhibiting effect.

The carnosol and carnosic acid used in the present invention are preferably in the form of a natural extract from the viewpoint of safety. Natural products of the carnosol and carnosic acid may be extracts obtained from herb-based plants such as sage, thyme and oregano and are preferably extracts obtained from rosemary containing a large amount of carnosol and carnosic acid.

The carnosol and carnosic acid may be obtained as a water-insoluble extract of rosemary. An example of the general production method of the carnosol and carnosic acid is as follows. First, similarly to the above water-soluble extracts, rosemary is subjected to extraction treatment with hexane, hexane/ethanol, ethanol, hydrated ethanol or supercritical carbon dioxide, and then water is added to the resultant extract to precipitate water-insoluble components therefrom. After the resultant mixture is mixed with activated carbon and then stirred, a mixture of the water-insoluble components and activated carbon is separated from the extract. The resultant mixture is further subjected to extract treatment with hexane, hexane/ethanol, ethanol, hydrated ethanol or supercritical carbon dioxide, and the obtained extract was distilled to remove the extraction solvent therefrom, thereby obtaining the carnosol and carnosic acid in the form of a powdered concentrate. The details of the above method for production of the carnosol and carnosic acid will become more apparent by referring to descriptions of Japanese Patent Publication (KOKOKU) No. 59-4469.

(Emulsifying agent)

As the emulsifying agent, there may be used any of conventional emulsifying agents which have been used in application fields of foods, diets, cosmetics, drugs or medicines, industrial products, etc. That is, the emulsifying agent means an amphiphatic compound containing both a hydrophilic group moiety and a hydrophobic group moiety therein which are chemically bonded to each other. The hydrophilic group moiety may be either ionic, nonionic or amphoteric, and further may be a hydrophilic polymer. The hydrophobic group moiety may be constituted from typically a fatty acid having usually 2 to 40 carbon atoms and preferably 6 to 24 carbon atoms. Also, the hydrophobic group moiety may be constituted from not fatty acid but cholesterol group or hydrophobic polymer.

Examples of the emulsifying agents for foods may include sucrose fatty esters, glycerin fatty esters, polyglycerin fatty esters, organic acid glycerin fatty esters, lactic acid fatty esters and sorbitan fatty esters. Examples of the emulsifying agents produced from natural products may include lecithins such as vegetable lecithin, yolk lecithin, fractional lecithin and enzyme-treated lecithin; saponins such as saponin, Quilaja saponin and soybean saponin; phospholipids such as sphingolipids, vegetable sterols and animal sterols; bile powder; and glycolipids such as tomato glycolipid.

In the anti-degradation agent of the present invention, the water-insoluble antiaxidant comprises carnosol and/or carnosic acid, whereas the water-soluble antioxidant used therein is preferably rosmarinic acid.

In the anti-degradation agent of the present invention, the amounts of the water-insoluble antioxidant, the water-soluble antioxidant and the emulsifying agent used therein vary depending upon kinds of the respective components used and, therefor, is not specifically determined. However, the lower limit of a total content of the carnosol and carnosic acid is not less than 4% by weight and becomes more preferred in the order of 5% by weight, 6% by weight, 8% by weight, 12% by weight and 20% by weight. On the other hand, the upper limit of the total content of the carnosol and carnosic acid is usually 95% by weight and preferably 80% by weight. Whereas, the lower limit of a content of the vitamin E is usually not less than 10% by weight, preferably 15% by weight, more preferably 18% by weight and still more preferably 20% by weight. On the other hand, the upper limit of the content of the vitamin E is usually 95% by weight and preferably 80% by weight. Further, the content of the water-soluble antioxidant is usually not less than 0.5% by weight which value is especially useful when the water-soluble antioxidant is rosmaric acid. In addition, the amount of the emulsifying agent used is usually 0.01 to 20% by weight and preferably 0.1 to 10% by weight on the basis of the total amount of the water-insoluble antioxidant and the water-soluble antioxidant.

The weight ratio of the content of the water-soluble antioxidant to the total content of the carnosol and carnosic acid is usually 10/1 to 1/99 and preferably 1/2 to 1/30. When the weight ratio is controlled to the above-specified range, the resultant anti-degradation agent can be prevented from being deteriorated in degradation-inhibiting performance thereof owing to external environments, and can also be enhanced in stability to light and heat. When the weight ratio of the water-soluble antioxidant is too small, the obtained anti-degradation agent tends to be deteriorated in oxidation-inhibiting performance at an interface between water and oil. On the other hand, when the weight ratio of the water-soluble antioxidant is too large, the obtained anti-degradation agent tends to be lowered in degradation-inhibiting performance for oils and fats.

(Other components)

The anti-degradation agent of the present invention may also contain other optional components, for example, those components mixed therein owing to the methods used for procuring rosmarinic acid, carnosol and carnosic acid. More specifically, the anti-degradation agent may contain optional components which may be extracted upon subjecting herbs to extraction treatment. Further, in addition to the above components, the anti-degradation agent may be used in combination with emulsifying agents, e.g., polyglycerin fatty esters such as polyglycerin lauric esters, polyglycerin myristic esters, polyglycerin palmitic esters, polyglycerin stearic esters and polyglycerin oleic esters, and sucrose fatty esters such as sucrose lauric esters, sucrose myristic esters, sucrose palmitic esters, sucrose stearic esters and sucrose oleic esters; and natural emulsifying agents such as lecithin, phospholipid, cholesterol and licorice. The content of these components is usually 1 to 40% by weight, preferably 3 to 30% by weight and more preferably 5 to 20% by weight.

Further, the anti-degradation agent of the present invention may be used in combination with the other antioxidants such as coffee bean extracts, sunflower extracts, grape seeds, $\alpha$G rutin, catechin and green tea extracts. These other antioxidants may further contain vitamin C, vitamin E (tocopherol), vitamin P and chlorogenic acid. The content of the other antioxidants is usually 0.1 to 50% by weight, preferably 0.5 to 30% by weight and more preferably 1 to 20% by weight. Further, the anti-degradation agent of the present invention may be used in combination with sugar alcohols such as "Oligotose", trehalose, xylitol and erythritol, and sugars. The content of the sugar alcohols or sugars is usually 0.1 to 50% by weight, preferably 0.5 to 30% by weight and more preferably 1 to 20% by weight.

The method for producing the anti-degradation agent of the present invention is not particularly limited, but is preferably the above extraction method in which herbs such as rosemary are used as the raw material. Also, there may be used the method of mixing different extracts obtained by varying extraction conditions with each other.

The anti-degradation agent of the present invention may be usually used in the form of a solution obtained by dissolving the above respective components in water or a mixed solvent of water and ethanol. When using carnosol and carnosic acid in combination, both the compounds are usually dissolved in the mixed solvent of water and ethanol. The solution of the carnosol and carnosic acid in the mixed solvent may be usually prepared by mixing the above respective components with each other, adding ethanol to the resultant mixture, and then adding water to the ethanol solution. The mixing ratio of water to ethanol in the mixed solvent is usually 1:1 to 3:1. The anti-degradation agent of the present invention may be in the form of a powder. Such a powder of the anti-degradation agent may be produced by spray-drying or freeze-drying the above solution.

The anti-degradation agent of the present invention may be suitably applied to foods and cosmetics which tend to be readily deteriorated in quality. In this case, the amount of the anti-degradation agent added is usually 0.0001 to 30% by weight, preferably 0.0003 to 10% by weight and more preferably 0.0005 to 5% by weight on the basis of the weight of the respective products.

<Method for evaluating performance of anti-degradation agent of the present invention>

The parameters defined with respect to the anti-degradation agent can also be used for the evaluation method thereof. That is, the photo resistance and the heat resistance of the anti-degradation agents can be evaluated according to the formulae (1) and (2) or the formulae (3) and (4).

The anti-degradation agent exhibits a photo resistance of not less than 10 and a heat resistance of not less than 10. Both of the photo resistance and the heat resistance of the anti-degradation agent are more preferably not less than 20.

<Products using the anti-degradation agent of the present invention>

(Beverage or food)

As the beverage or food of the present invention, there may be suitably used foods which tend to be readily deteriorated in quality. Specific examples of the beverage or food may include beverages, milk beverages, alcohol beverages, boiled rice, beans (such as rice, wheat, barley, corn, millet and barnyard millet), bread and other wheat flour products, noodles, roux such as curry roux and stew roux, frozen foods, chilled foods, retort foods, dairy products such as ice cream, milk-processed foods, beverages such as milk, soft drinks, carbonated beverages, green teas, black teas, oolong teas, coffees, cocoas, refined sake, beers, sparkling wines, synthetic refined sake, sweet sake (mirin), wines, shoutu, whiskies and vegetable juices, condiments such as miso, soy sauce, vinegar, taste seasoning, dressing, sauce and mayonnaise, processed marine products such as fish paste products, fish ham and sausage, dried bonito and foods boiled down in soy, frozen foods such as frozen boiled rice, frozen noodles, frozen croquette, frozen hamburger, frozen shao-mai, frozen gyoza and frozen gratin, instant foods such as instant noodles, instant soups, instant curry, instant miso soups and instant coffees, and confectioneries such as Japanese-style cakes, Japanese unbaked cakes, Japanese semi-baked cakes, baked cakes, unbaked cakes, semi-baked cakes, candies, chocolates, chewing gums, biscuits, rice cakes, snack cakes, oil cakes and other cakes.

Among the above beverages and foods, the processed marine, livestock or oil and fat products are preferably those products which tend to be readily deteriorated in quality or should be preserved for a long period of time. Specific examples of the processed marine, livestock or oil and fat products may include fresh fishes, dried fishes, overnight-dried fishes, dried mirin-seasoned fishes, pigment-keeping agents for shells, red-meat fishes and Crustacea, minced or ground fish meat, marine paste products, foods of delicate flavor, fish sausage, salt-preserved products, laver, seaweed products, unsaturated polyvalent fatty acids such as $\alpha$-linolenic acid, docosahexaenoic acid (DHA) and eicosapentaenoic acid (EPA) and triglycerides thereof as well as foods, chicken, pork, beef, mutton, sausage, ham and their processed products containing these compounds, corn flakes, instant Chinese noodles, oil cakes using oils and fats, fast spreads, and margarine.

The amount of the anti-degradation agent used in the beverage or food is 0.0001 to 30% by weight, preferably 0.0003 to 10% by weight and more preferably 0.0005 to 5% by weight on the basis of the weight of the beverage or food.

The anti-degradation agent for the beverage or food is preferably constituted from carnosol and/or carnosic acid. The amount of the carnosol and/or carnosic acid added is usually not less than 0.5 ppm, preferably not less than 5 ppm, more preferably not less than 40 ppm and especially preferably not less than 100 ppm on the basis of the amount of the beverage or food. The upper limit of the amount of the carnosol and/or carnosic acid added is usually 10,000 ppm.

Further, in the anti-degradation agent for the beverage or food, rosmarinic acid is preferably used in combination with the carnosol and/or carnosic acid. The amount of the rosmarinic acid added to the beverage or food is usually not less than 5 ppm, preferably not less than 50 ppm, more preferably not less than 500 ppm and especially preferably not less than 1,000 ppm on the basis of the amount of the beverage or food. The upper limit of the amount of the rosmarinic acid added is usually 100,000 ppm.

(Diet and pet food)

The anti-degradation agent of the present invention may also be applied to diets for livestock and cultured fishes as well as pet foods.

The amount of the anti-degradation agent used in the diet or pet food is 0.0001 to 30% by weight, preferably 0.0003 to 30% by weight and more preferably 0.0005 to 30% by weight on the basis of the weight of the diet or pet food.

The anti-degradation agent for the diet or pet food is preferably constituted from carnosol and/or carnosic acid. The amount of the carnosol and/or carnosic acid added is usually not less than 0.5 ppm, preferably not less than 5 ppm, more preferably not less than 40 ppm and especially preferably not less than 100 ppm on the basis of the amount of the diet or pet food. The upper limit of the amount of the carnosol and/or carnosic acid added is usually 100,000 ppm.

Further, in the anti-degradation agent for the diet or pet food, rosmarinic acid is preferably used in combination with the carnosol and/or carnosic acid. The amount of the rosmarinic acid added is usually not less than 5 ppm, preferably not less than 50 ppm, more preferably not less than 500 ppm and especially preferably not less than 1,000 ppm on the basis of the amount of the diet or pet food. The upper limit of the amount of the rosmarinic acid added is usually 100,000 ppm.

(Perfumes or cosmetic)

As the perfume or cosmetic usable in the present invention, there may be suitably used those perfumes and cosmetics which tend to be readily deteriorated in quality. Specific examples of the perfumes and cosmetics may include humectants, beauty white agents, cleansing solutions, lotions, detergents, softening agents, finishing agents, dish-washing agents, detergents for vegetables and fruits and rinsing agents. The amount of the anti-degradation agent used is 0.0001 to 30% by weight, preferably 0.0003 to 10% by weight and more preferably 0.0005 to 5% by weight on the basis of the weight of the perfume or cosmetic.

(Glaze agent)

The term "glaze" means to coat the surface of landed fish with ice upon freezing the landed fish, and the glaze agent is used for forming a uniform ice coat on the surface of the landed fish. Examples of the glaze agent may include functional water, electrolytic water and UV-treated water. The amount of the anti-degradation agent used in the glaze agent is 0.0001 to 30% by weight, preferably 0.0003 to 10% by weight and more preferably 0.0005 to 5% by weight on the basis of the weight of the glaze agent.

(Plastic product)

The anti-degradation agent of the present invention can be added to a plastic product to indirectly prevent beverage or food, perfume or cosmetic and other products enclosed therein from being deteriorated in quality. Specific examples of the plastic product may include plastic containers for the beverage or food and the perfume or cosmetic, packaging materials for foods such as baran

(aspidistra) partitions and packs for preservation of cooked foods, packaging materials for sanitary goods such as deodorants and liquid detergents, white home appliances such as refrigerators, air conditioners, air cleaners and laundry/drying machines, and air conditioning equipments for ships, automobiles, trains, airplanes and buildings. The amount of the anti-degradation agent used in the plastic product is 0.00001 to 20% by weight, preferably 0.0001 to 10% by weight and more preferably 0.0005 to 5% by weight on the basis of the weight of the plastic product.

EXAMPLES

The present invention is described in more detail by Examples. However, it should be noted that the following Examples are only illustrative and not intended to limit the scope of the present invention.

Production Example 1:

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was repeated two times, thereby obtaining filtrates. The obtained filtrates were combined together and mixed with 5 L of water to form a precipitate. The resultant solution was mixed with 100 g of activated carbon, stirred for 1 hr, allowed to stand in a cold place overnight, and then filtered to obtain a filtrate. The thus obtained filtrate was concentrated under reduced pressure, thereby obtaining 120 g of a (water-soluble) rosemary extract (1). As a result, it was confirmed that the rosemary extract (1) had a rosmarinic acid content of 31.6% by weight.

Production Example 2:

10 L of 50% hydrated ethanol was added to 1 kg of rosemary, and the resultant mixture was refluxed under heating for 3 hr and then filtered under a hot condition, thereby obtaining a filtrate. The obtained residue was extracted with 6 L of 50% hydrated ethanol, and this extraction procedure was repeated two times, thereby obtaining filtrates. The obtained filtrates were combined together and mixed with 5 L of water to form a precipitate. The resultant filtrate was mixed with 100 g of activated carbon, stirred for 1 hr, allowed to stand in a cold place overnight, and then filtered to obtain a mixture of a precipitate and activated carbon. The thus obtained mixture was mixed with 4 L of ethanol, refluxed under heating for 3 hr, and then filtered under a hot condition, thereby obtaining a filtrate. The obtained residue was extracted with 2.4 L of ethanol, and this extraction procedure was repeated two times, thereby obtaining filtrates. The obtained filtrates were combined together and concentrated under reduced pressure to distill off ethanol therefrom, thereby obtaining a (water-insoluble) powdery rosemary extract (2). As a result, it was confirmed that the rosemary extract (2) had a total content of carnosol and carnosic acid of 24.9% by weight.

Production Example 3:

10 L of ethanol was added to 1 kg of rosemary, and the resultant mixture was refluxed under heating for 3 hr and then filtered under a hot condition, thereby obtaining a filtrate. The obtained residue was extracted with 6 L of ethanol, and this extraction procedure was repeated two times, thereby obtaining filtrates. The obtained filtrates were combined together and mixed with 100 g of activated carbon, stirred for 1 hr, allowed to stand in a cold place overnight, and then filtered, thereby obtaining a rosemary extract (3). As a result, it was confirmed that the rosemary extract (3) had a rosmarinic acid content of 0.25% by weight and a total content of carnosol and carnosic acid of 2.9% by weight.

Examples 1 to 3 and Comparative Examples 1 to 3:

<Production of kneaded material>

A kneaded material composed of 30 parts by weight of a soybean oil, 50 parts by weight of a wheat flour and 20 part by weight of water was subjected to deterioration test.
More specifically, the respective anti-degradation agent samples shown in Table 2 were added to a mixture composed of 30 parts by weight of a soybean oil, 50 parts by weight of a wheat flour and 20 part by weight of water in a total amount of 0.1% by weight based on the weight of the mixture (at a mixing ratio shown in Table 2), and the resultant mixture was kneaded together by hands until a uniform kneaded material was produced. The heat resistance (oxidation odor-preventing property) of the thus produced kneaded material was evaluated according to the above formula (2). The results are shown in Table 2. During the evaluation test, the kneaded material was visually observed to determine a change in color (brown discoloration-preventing property) thereof. The evaluation criteria are shown in Table 1 below. The results are shown in Table 2.

Table 1

| Color of kneaded material | Brown discoloration-preventing property |
|---|---|
| Similar to color upon no addition | 0 |
| Slightly lighter than color upon no addition | 20 |
| Apparently lighter than color upon no addition | 50 |
| Slightly tinted | 80 |

(continued)

| Color of kneaded material | Brown discoloration-preventing property |
|---|---|
| Not tinted | 100 |

Table 2

| | Examples | | | Comparative Examples | | |
|---|---|---|---|---|---|---|
| | 1 | 2 | 3 | 1 | 2 | 3 |
| Rosemary extract (1) (wt%) | 80 | 50 | 5 | 0 | 100 | 0 |
| Rosemary extract (2) (wt%) | 20 | 50 | 95 | 0 | 0 | 100 |
| Rosemary extract (3) (wt%) | 0 | 0 | 0 | 100 | 0 | 0 |
| Rosmarinic acid (A) (wt%) | 25.3 | 15.8 | 1.6 | 0.25 | 31.6 | 0 |
| Carnosol + carnosic acid {(B) + (C)} (wt%) | 4.98 | 12.5 | 24 | 2.9 | 0 | 24.9 |
| {(B) + (C)} / (A) | 0.2 | 0.79 | 15 | 11.6 | (100/0) | 0 |
| Evaluation | | | | | | |
| Oxidation odor-preventing property | 10 | 18 | 20 | -1 | 4 | 22 |
| Brown discoloration-preventing property | 100 | 100 | 80 | 50 | 100 | 50 |
| Total evaluation | B | B | A | C | C | C |

In Table 2, the "total evaluation" was conducted using ratings shown in Table 3 below.

Table 3

| A | Oxidation odor-preventing property of not less than 20 and a brown discoloration-preventing property of not less than 80 (highly effective) |
|---|---|
| B | Oxidation odor-preventing property of not less than 10 and a brown discoloration-preventing property of not less than 80 (effective) |
| C | Other than the above cases (ineffective) |

From the above results, it has been apparently recognized that the anti-degradation agents of the present invention were able to prevent generation of oxidation odor due to deterioration as well as brown discoloration due to heat.

Example 4:

A kneaded material composed of 30 parts by weight of a soybean oil, 50 parts by weight of a wheat flour and 20 part by weight of water was subjected to deterioration test. More specifically, the respective anti-degradation agent samples shown in Table 4 were added to a mixture composed of 30 parts by weight of a soybean oil, 50 parts by weight of a wheat flour and 20 part by weight of water in a total amount of 0.1% by weight based on the weight of the mixture, and the resultant mixture was mechanically kneaded together to obtain a kneaded material. The photo resistance and the heat resistance of the thus obtained kneaded material was evaluated according to the above formulae (1) and (2), respectively. The results are shown in Table 4.

Table 4

| Anti-degradation agent sample | Photo resistance | Heat resistance |
|---|---|---|
| Mixture of 80 parts by weight of rosemary extract (1) and 20 parts by weight of rosemary extract (2) | 80 | 12 |
| Mixture of 50 parts by weight of rosemary extract (1) and 50 parts by weight of rosemary extract (2) | 75 | 18 |

(continued)

| Anti-degradation agent sample | Photo resistance | Heat resistance |
|---|---|---|
| Mixture of 5 parts by weight of rosemary extract (1) and 95 parts by weight of carnosol | 90 | 20 |
| Rosemary extract (3)* | 2 | -1 |
| Rosemary extract (1)* | 4 | 4 |
| Rosemary extract (2)* | 8 | 22 |
| No addition* | 0 | 0 |
| * : For comparison only; not part of the present invention | | |

Example 5:

A red radish pigment was subjected to deterioration test. More specifically, 0.1% of the red radish pigment was added to a 7% alcohol (ethanol) aqueous solution, and the respective anti-degradation agent samples shown in Table 5 were further added to the solution in a total amount of 0.1% by weight. The thus obtained sample solution was irradiated with light at a total illuminance of 500,000 lux (i.e., light irradiation: 20000 lux x 25 hr) (at 5°C) to evaluate a photo resistance thereof, and allowed to stand at 55°C for one week to evaluate a heat resistance thereof. The degree of discoloration of the pigment was evaluated by measuring an absorbance thereof at a specific wavelength. The photo resistance and the heat resistance were evaluated according to the above formulae (3) and (4), respectively, and expressed by the relative values obtained when each of the photo resistance and heat resistance before the test was regarded as 100. The results are shown in Table 5

Table 5

| Anti-degradation agent sample | Photo resistance | Heat resistance |
|---|---|---|
| Rosemary extract (1)* | 2 | 5 |
| Mixture of 67 parts by weight of rosemary extract (1) and 33 parts by weight of rosemary extract (2) | 23 | 17 |
| Mixture of 40 parts by weight of rosemary extract (1), 20 parts by weight of rosemary extract (2) and 40 parts by weight of polyglycerin monostearic ester | 29 | 19 |
| * : For comparison only; not part of the present invention | | |

Example 6:

A kneaded material composed of 30 parts by weight of a lard (animal oil), 50 parts by weight of a wheat flour and 20 part by weight of water was subjected to deterioration test. More specifically, the respective anti-degradation agent samples shown in Table 6 were added to a mixture composed of 30 parts by weight of a lard (animal oil), 50 parts by weight of a wheat flour and 20 part by weight of water in a total amount of 0.1% by weight based on the weight of the mixture, and the resultant mixture was mechanically kneaded together to obtain a kneaded material. Then, a sampling tube was charged with 10 g of the thus obtained kneaded material, air was blown into the sampling tube, and then the material was held at 60°C to evaluate a heat resistance thereof by the same method as defined above. The results are shown in Table 6.

Table 6

| Anti-degradation agent sample | Heat resistance |
|---|---|
| Mixture of 16 parts by weight of rosemary extract (2), 16 parts by weight of vitamin C and 64 parts by weight of polyglycerin dodecaerucic ester | 27 |
| Mixture of 16 parts by weight of rosemary extract (1), 16 parts by weight of rosemary extract (2) and 64 parts by weight of polyglycerin dodecaerucic ester | 22 |

(continued)

| Anti-degradation agent sample | Heat resistance |
|---|---|
| Mixture of 16 parts by weight of vitamin C, 16 parts by weight of mixed tocopherol produced by Amakasu Co., Ltd., and 64 parts by weight of polyglycerin dodecaerucic ester* | 18 |
| Mixture of 16 parts by weight of rosemary extract (1), 16 parts by weight of vitamin C and 64 parts by weight of polyglycerin dodecaerucic ester For comparison only; not part of the present invention | 3 |
| Mixture of 6 parts by weight of vitamin C, 64 parts by weight of polyglycerin dodecaerucic ester and 16 parts by weight of water* | 8 |
| Mixture of 16 parts by weight of rosemary extract (1), 64 parts by weight of polyglycerin dodecaerucic ester and 16 parts by weight of water* | 2 |
| * : For comparison only; not part of the present invention | |

Comparative Example 4 (For comparison only; not part of the present invention)

A pet food was subjected to deterioration test. More specifically, oils and fats and chicken meal to which the respective anti-degradation agents shown in Table 7 and vitamin E were added, were mixed with each other, and the resultant mixture was molded into tablets of pet food using a tableting machine. The amount of the anti-degradation agents added was 2 ppm. Then, a sampling tube was charged with 10 g of the thus obtained pet food, air was blown into the sampling tube, and then the pet food was held at 110°C to evaluate a heat resistance thereof by the same method as defined above. The results are shown in Table 7.

Separately, the rosemary extract (2) was added to the pet food, and 100 of the pet food was extracted with 1 L of THF to obtain an extract. The thus obtained extract was subjected to high-speed liquid chromatography for analysis and evaluation thereof. As a result, it was confirmed that a total amount of carnosol and carnosic acid contained in the extract was 0.5 ppm.

Table 7

| Anti-degradation agent sample | Heat resistance |
|---|---|
| Rosemary extract (2) | 12.7 |
| Rosemary extract (3) | 4.9 |

**Claims**

1. An anti-degradation agent which comprises:

- a water-insoluble antioxidant having a solubility in 100 g of water of less than 0.1 g at 25 °C, wherein the water insoluble antioxidant comprises carnosol and/or carnosic acid and the total amount of the carnosol and/or carnosic acid in the anti-degradation agent is not less than 4% by weight; and
- a water-soluble antioxidant having a solubility in 100 g of water of not less than 0.1 g at 25 °C;

which anti-degradation agent has a photo resistance of not less than 10 and a heat resistance of not less than 10 wherein the photo resistance is determined as in definition (A) and the heat resistance as in definition (B) or the photo resistance is determined as in definition (C) and the heat resistance as in definition (D);

(A) When a food as a sample is placed in a container and irradiated with light at a total illuminance of 500,000 lux to measure an amount of hexanal generated from the food and accumulated within a head space of the container by gas chromatography, said photo resistance being calculated from the following formula (1):

Photo resistance = [ {(Amount of hexanal generated from the light-irradiated food containing no anti-degradation agent) – (Amount of hexanal generated from the light-irradiated food containing the anti-degradation agent)}/{(Amount of hexanal generated from the light-irradiated food containing no anti-degradation agent) – (Amount of hexanal generated from the light-non-irradiated food containing the anti-degradation agent)}] x 100 ⋯ (1);

(B) When a food as a sample is placed in a container, air is blown into the container, and the food is maintained at a temperature of 60°C to measure a time at which an amount of volatile components generated from the food is rapidly increased (degradation induction time) using an electrical conductivity meter, said heat resistance being calculated from the following formula (2):

Heat Resistance = [ {(Degradation induction time of the food containing the anti-degradation agent upon heating) – (Degradation induction time of the food containing no anti-degradation agent upon heating)}/(Degradation induction time of the food containing no anti-degradation agent upon heating)] x 100 ⋯ (2);

(C) When a solution of a pigment as a sample filled in a container is irradiated with light at a total illuminance of 500,000 lux to determine a degree of discoloration of the pigment by measuring an absorbance of the pigment solution at a specific wavelength, said photo resistance being calculated from the following formula (3):

Photo resistance = [ {(Absorbance of the light-irradiated pigment solution containing the anti-degradation agent) – (Absorbance of the light-irradiated pigment solution containing no anti-degradation agent)}/{(Absorbance of the light-non-irradiated pigment solution containing the anti-degradation agent) – (Absorbance of the light-irradiated pigment solution containing no anti-degradation agent)}] x 100 ⋯ (3);

and
(D) When a pigment as a sample placed in a container is held at 55 °C for one week to determine a degree of discoloration of the pigment by measuring an absorbance of a solution of the pigment at a specific wavelength, said heat resistance being calculated from the following formula (4):

Heat Resistance = [ {(Absorbance of the heated pigment solution containing the anti-degradation agent) – (Absorbance of the heated pigment solution containing no anti-degradation agent)}/{(Absorbance of the non-heated pigment solution containing the anti-degradation agent) – (Absorbance of the heated pigment solution containing no anti-degradation agent)}] x 100 ⋯ (4).

2. An anti-degradation agent according to claim 1, wherein the water-soluble antioxidant is rosmarinic acid.

3. An anti-degradation agent according to claim 2, wherein the rosmarinic acid is contained in an amount of not less than 0.5% by weight.

4. An anti-degradation agent according to claim 2 or 3, wherein a weight ratio of the rosmarinic acid to the sum of the carnosol and the carnosic acid is 10/1 to 1/99.

5. An anti-degradation agent according to any one of claims 1 to 4, which further comprises an emulsifying agent.

6. A beverage or food comprising the anti-degradation agent as defined in any one of claims 1 to 5 in an amount of 0.0001 to 30% by weight.

7. A diet or pet food comprising the anti-degradation agent as defined in any one of claims 1 to 5 in an amount of 0.0001 to 30% by weight.

8. A perfume or cosmetic comprising the anti-degradation agent as defined in any one of claims 1 to 5 in an amount of 0.0001 to 30% by weight.

9. A glaze agent comprising the anti-degradation agent as defined in any one of claims 1 to 5 in an amount of 0.0001 to 30% by weight.

10. A plastic product comprising the anti-degradation agent as defined in any one of claims 1 to 5 in an amount of 0.00001 to 20% by weight.

11. A process which comprises applying to a beverage or food the anti-degradation agent as defined in any one of claims 1 to 5.

12. A process which comprises applying to a diet or pet food the anti-degradation agent as defined in any one of claims 1 to 5.

13. A process which comprises applying to a perfume or cosmetic the anti-degradation agent as defined in any one of claims 1 to 5.

14. A process which comprises applying to a glaze agent the anti-degradation agent as defined in any one of claims 1 to 5.

15. A process which comprises applying to a plastic product the anti-degradation agent as defined in any one of claims 1 to 5.

16. A process as defined in any one of claims 11 to 14 wherein the anti-degradation agent is applied in an amount of 0.0001 to 30% by weight.

17. A process as defined in claim 15 wherein the anti-degradation agent is applied in an amount of 0.00001 to 20% by weight.


**Patentansprüche**

1. Anti-Abbau-Mittel, das umfasst:

- ein in Wasser unlösliches Antioxidans, das eine Löslichkeit in 100 g Wasser von weniger als 0,1 g bei 25°C hat, wobei das in Wasser unlösliche Antioxidans Carnosol und/oder Carnosolsäure umfasst und die Gesamtmenge an Carnosol und/oder Carnosolsäure in dem Anti-Abbau-Mittel nicht weniger als 4 Gew.-% ist, und
- ein wasserlösliches Antioxidans, das eine Löslichkeit in 100 g Wasser von nicht weniger als 0,1 g bei 25°C hat;

wobei das Anti-Abbau-Mittel einen Photowiderstand von nicht kleiner als 10 und eine Wärmestabilität von nicht kleiner als 10 hat, wobei der Photowiderstand wie in Definition (A) und die Wärmestabilität wie in Definition (B) bestimmt wird oder der Photowiderstand wie in Definition (C) und die Wärmestabilität wie in Definition (D) bestimmt wird;

(A) wenn ein Nahrungsmittel als Probe in einen Behälter gegeben wird und mit Licht mit einer gesamten Beleuchtungsstärke von 500.000 Lux bestrahlt wird, um die Menge an Hexanal, die von dem Nahrungsmittel erzeugt wird und im Kopfraum des Behälters akkumuliert wird, durch Gaschromatographie zu messen, wird der Photowiderstand aus der folgenden Gleichung (1) errechnet:

Photowiderstand = [{(Menge an Hexanal, erzeugt von dem mit Licht bestrahlten Nahrungsmittel, das kein Anti-Abbau-Mittel enthält) – (Menge an Hexanal, erzeugt von dem mit Licht bestrahlten Nahrungsmittel, das das Anti-Abbau-Mittel enthält)}/{(Menge an Hexanal, erzeugt von dem mit Licht bestrahlten Nahrungsmittel, das kein Anti-Abbau-Mittel enthält) – (Menge an Hexanal, erzeugt von dem nicht mit Licht bestrahlten Nahrungsmittel, das das Anti-Abbau-Mittel enthält)}] x 100 ··· (1);

(B) wenn ein Nahrungsmittel als Probe in einen Behälter gegeben wird, Luft in den Behälter geblasen wird und das Nahrungsmittel bei einer Temperatur von 60°C gehalten wird, um die Zeit zu messen, bei der die Menge an flüchtigen Komponenten, die von dem Nahrungsmittel erzeugt werden, rasch angestiegen ist (Abbauinduktionszeit), wobei ein Messgerät für die elektrische Leitfähigkeit verwendet wird, die Wärmestabilität mit der folgenden Gleichung (2) errechnet wird:

Wärmestabilität = [{(Abbauinduktionszeit des Nahrungsmittels, das das Anti-Abbau-Mittel enthält, bei Erwärmen) – (Abbauinduktionszeit des Nahrungsmittels, das kein Anti-Abbau-Mittel enthält, bei Erwärmen)}/(Abbauinduktionszeit des Nahrungsmittels, das kein Anti-Abbau-Mittel enthält, bei Erwärmen)] x 100 ··· (2);

(C) wenn eine Lösung eines Pigments als Probe, die in einen Behälter gefüllt ist, mit Licht mit einer gesamten Beleuchtungsstärke von 500.000 Lux bestrahlt wird, um den Grad der Verfärbung des Pigments zu bestimmen, indem die Extinktion der Pigmentlösung bei einer spezifischen Wellenlänge gemessen wird, kann der Photowiderstand aus der folgenden Gleichung (3) errechnet werden:

Photowiderstand = [{(Extinktion der mit Licht bestrahlten Pigmentlösung, die das Anti-Abbau-Mittel enthält) – (Extinktion der mit Licht bestrahlten Pigmentlösung, die kein Anti-Abbau-Mittel enthält)}/{(Extinktion der nicht mit Licht bestrahlten Pigmentlösung, die das Anti-Abbau-Mittel enthält) – (Extinktion der mit Licht bestrahlten Pigmentlösung, die kein Anti-Abbau-Mittel enthält)}] x 100 ··· (3),

und
(D) wenn ein Pigment als Probe, das in einen Behälter gegeben wurde, für eine Woche bei 55°C gehalten wird, um den Verfärbungsgrad des Pigments durch Messung der Extinktion einer Lösung des Pigments bei einer spezifischen Wellenlänge zu bestimmen, wird die Wärmestabilität aus der folgenden Gleichung (4) errechnet:

```
Wärmestabilität    =    [{(Extinktion    der    erwärmten
Pigmentlösung,   die   das   Anti-Abbau-Mittel   enthält)   -
(Extinktion der erwärmten Pigmentlösung, die kein Anti-Abbau-
Mittel    enthält)}/{(Extinktion    der    nicht    erwärmten
Pigmentlösung,   die   das   Anti-Abbau-Mittel   enthält)   -
(Extinktion der erwärmten Pigmentlösung, die kein Anti-Abbau-
Mittel enthält)}] x 100 ··· (4).
```

2. Anti-Abbau-Mittel gemäß Anspruch 1, wobei das wasserlösliche Antioxidans Rosmarinsäure ist.

3. Anti-Abbau-Mittel gemäß Anspruch 2, wobei die Rosmarinsäure in einer Menge von nicht weniger als 0,5 Gew.-% enthalten ist.

4. Anti-Abbau-Mittel gemäß Anspruch 2 oder 3, wobei das Gewichtsverhältnis der Rosmarinsäure zu der Summe aus Carnosol und Carnosolsäure 10/1 bis 1/99 ist.

5. Anti-Abbau-Mittel gemäß einem der Ansprüche 1 bis 4, das außerdem ein Emulgiermittel umfasst.

6. Getränk oder Nahrungsmittel, das das Anti-Abbau-Mittel, wie es in einem der Ansprüche 1 bis 5 definiert ist, in einer Menge von 0,0001 bis 30 Gew.-% umfasst.

7. Diät- oder Tiernahrungsmittel, das das Anti-Abbau-Mittel, wie es in einem der Ansprüche 1 bis 5 definiert ist, in einer Menge von 0,0001 bis 30 Gew.-% umfasst.

8. Parfum oder Kosmetikum, das das Anti-Abbau-Mittel, wie es in einem der Ansprüche 1 bis 5 definiert ist, in einer Menge von 0,0001 bis 30 Gew.-% umfasst.

9. Glasur, die das Anti-Abbau-Mittel, wie es in einem der Ansprüche 1 bis 5 definiert ist, in einer Menge von 0,0001 bis 30 Gew.-% umfasst.

10. Kunststoffprodukt, das das Anti-Abbau-Mittel, wie es in einem der Ansprüche 1 bis 5 definiert ist, in einer Menge von 0,00001 bis 20 Gew.-% umfasst.

11. Verfahren, das Anwenden des Anti-Abbau-Mittels, wie es in einem der Ansprüche 1 bis 5 definiert ist, auf ein Getränk oder Nahrungsmittel umfasst.

12. Verfahren, das Anwenden des Anti-Abbau-Mittels, wie es in einem der Ansprüche 1 bis 5 definiert ist, auf ein Diät- oder Tiernahrungsmittel umfasst.

13. Verfahren, das Anwenden des Anti-Abbau-Mittels, wie es in einem der Ansprüche 1 bis 5 definiert ist, auf ein Parfum oder Kosmetikum umfasst.

14. Verfahren, das Anwenden es Anti-Abbau-Mittels, wie es in einem der Ansprüche 1 bis 5 definiert ist, auf eine Glasur umfasst.

15. Verfahren, das Anwenden des Anti-Abbau-Mittels, wie es in einem der Ansprüche 1 bis 5 definiert ist, auf ein Kunststoffprodukt umfasst.

16. Verfahren, wie es in einem der Ansprüche 11 bis 14 definiert ist, wobei das Anti-Abbau-Mittel in einer Menge von 0,0001 bis 30 Gew.-% angewendet wird.

**17.** Verfahren, wie es in Anspruch 15 definiert ist, wobei das Anti-Abbau-Mittel in einer Menge von 0,00001 bis 20 Gew.-% angewendet wird.

**Revendications**

**1.** Inhibiteur de dégradation, qui comprend :

- un anti-oxydant insoluble dans l'eau, dont la solubilité vaut moins de 0,1 g dans 100 g d'eau à 25 °C, lequel antioxydant insoluble dans l'eau comprend du carnosol et/ou de l'acide carnosique, en une quantité totale de carnosol et/ou d'acide carnosique qui représente au moins 4 % du poids de l'inhibiteur de dégradation,
- et un anti-oxydant soluble dans l'eau, dont la solubilité vaut au moins 0,1 g dans 100 g d'eau à 25 °C,

lequel inhibiteur de dégradation présente un indice de résistance à la lumière d'au moins 10 et un indice de résistance à la chaleur d'au moins 10,
étant entendu que l'indice de résistance à la lumière est déterminé selon le protocole (A) ci-dessous et l'indice de résistance à la chaleur est déterminé selon le protocole (B) ci-dessous, ou bien que l'indice de résistance à la lumière est déterminé selon le protocole (C) ci-dessous et l'indice de résistance à la chaleur est déterminé selon le protocole (D) ci-dessous :

(A) on place un échantillon d'un aliment dans un récipient, on l'irradie avec de la lumière à un niveau d'éclairement total de 500 000 lux, et l'on mesure par chromatographie en phase gazeuse la quantité d'hexanal émise par l'aliment et accumulée dans l'espace de tête du récipient, puis on calcule ledit indice de résistance à la lumière par la formule (1) suivante :

$$\text{indice de résistance à la lumière} =$$
$$[\{(\text{quantité d'hexanal émise par l'aliment sans inhibiteur de dégradation et photo-irradié}) - (\text{quantité d'hexanal émise par l'aliment avec inhibiteur de dégradation et photo-irradié})\} / \{(\text{quantité d'hexanal émise par l'aliment sans inhibiteur de dégradation et photo-irradié}) - (\text{quantité d'hexanal émise par l'aliment avec inhibiteur de dégradation et non photo-irradié})\}] \times 100 \ \ldots\ (1) ;$$

(B) on place un échantillon d'un aliment dans un récipient, on souffle de l'air dans le récipient tout en maintenant l'aliment à la température de 60 °C et l'on mesure, à l'aide d'un appareil de mesure de la conductivité électrique, le temps au bout duquel la quantité de composants volatils émise par l'aliment augmente brusquement (temps d'induction de dégradation), puis on calcule ledit indice de résistance à la chaleur par la formule (2) suivante :

$$\text{indice de résistance à la chaleur} =$$
$$[\{(\text{temps d'induction de dégradation pour l'aliment avec inhibiteur de dégradation et chauffé}) - (\text{temps d'induction de dégradation pour l'aliment sans inhibiteur de dégradation et chauffé})\} / (\text{temps d'induction de dégradation pour l'aliment sans inhibiteur de dégradation et chauffé})] \times 100 \ \ldots\ (2) ;$$

(C) on remplit un récipient avec un échantillon d'une solution de pigment, on irradie cet échantillon avec de la lumière à un niveau d'éclairement total de 500 000 lux, et l'on détermine le degré de décoloration du pigment en mesurant l'absorbance de la solution de pigment à une longueur d'onde spécifique, puis on calcule ledit indice de résistance à la lumière par la formule (3) suivante :

$$indice\ de\ résistance\ à\ la\ lumière =$$

[{(absorbance de la solution de pigment avec inhibiteur de dégradation et photo-irradiée) − (absorbance de la solution de pigment sans nhibiteur de dégradation et photo-irradiée)} / {(absorbance de la solution de pigment avec inhibiteur de dégradation et non photo-irradiée) − (absorbance de la solution de pigment sans inhibiteur de dégradation et photo-irradiée)}] x 100 ..... (3) ;

(D) on place un échantillon de pigment dans un récipient, on le maintient à la température de 55 °C pendant une semaine, et l'on détermine le degré de décoloration du pigment en mesurant l'absorbance d'une solution du pigment à une longueur d'onde spécifique, puis on calcule ledit indice de résistance à la chaleur par la formule (4) suivante :

$$indice\ de\ résistance\ à\ la\ chaleur =$$

[{(absorbance de la solution de pigment avec inhibiteur de dégradation et chauffé) − (absorbance de la solution de pigment sans inhibiteur de dégradation et chauffé)} / {(absorbance de la solution de pigment avec inhibiteur de dégradation et non chauffé) − (absorbance de la solution de pigment sans inhibiteur de dégradation et chauffé)}] x 100 ..... (4).

**2.** Inhibiteur de dégradation conforme à la revendication 1, dans lequel l'anti-oxydant soluble dans l'eau est de l'acide romarinique.

**3.** Inhibiteur de dégradation conforme à la revendication 2, dans lequel l'acide romarinique se trouve présent en une proportion d'au moins 0,5 % en poids.

**4.** Inhibiteur de dégradation conforme à la revendication 2 ou 3, dans lequel le rapport du poids d'acide romarinique à la somme des poids de carnosol et d'acide carnosique vaut de 10/1 à 1/99.

**5.** Inhibiteur de dégradation conforme à l'une des revendications 1 à 4, qui comprend en outre un agent émulsifiant.

**6.** Boisson ou aliment comprenant un inhibiteur de dégradation conforme à l'une des revendications 1 à 5, en une proportion de 0,0001 à 30 % en poids.

**7.** Aliment de régime ou nourriture pour animaux familiers, comprenant un inhibiteur de dégradation conforme à l'une des revendications 1 à 5, en une proportion de 0,0001 à 30 % en poids.

**8.** Parfum ou produit cosmétique comprenant un inhibiteur de dégradation conforme à l'une des revendications 1 à 5, en une proportion de 0,0001 à 30 % en poids.

**9.** Agent de glaçage comprenant un inhibiteur de dégradation conforme à l'une des revendications 1 à 5, en une proportion de 0,0001 à 30 % en poids.

**10.** Produit en plastique comprenant un inhibiteur de dégradation conforme à l'une des revendications 1 à 5, en une proportion de 0,00001 à 20 % en poids.

**11.** Procédé qui comporte le fait d'introduire dans une boisson ou un aliment un inhibiteur de dégradation conforme à l'une des revendications 1 à 5.

**12.** Procédé qui comporte le fait d'introduire dans un aliment de régime ou de la nourriture pour animaux familiers un inhibiteur de dégradation conforme à l'une des revendications 1 à 5.

**13.** Procédé qui comporte le fait d'introduire dans un parfum ou un produit cosmétique un inhibiteur de dégradation conforme à l'une des revendications 1 à 5.

**14.** Procédé qui comporte le fait d'introduire dans un agent de glaçage un inhibiteur de dégradation conforme à l'une des revendications 1 à 5.

**15.** Procédé qui comporte le fait d'introduire dans un produit en plastique un inhibiteur de dégradation conforme à l'une des revendications 1 à 5.

**16.** Procédé conforme à l'une des revendications 11 à 14, dans lequel l'inhibiteur de dégradation est introduit en une proportion de 0,0001 à 30 % en poids.

**17.** Procédé conforme à la revendication 15, dans lequel l'inhibiteur de dégradation est introduit en une proportion de 0,00001 à 20 % en poids.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5795609 A
- JP 8253764 B
- JP 2002363557 A
- JP 2003055686 A
- JP 59004469 A